(19) Europäisches Patentamt
European Patent Office
Office européen des brevets

(11) EP 1 961 751 B1

(12) **EUROPEAN PATENT SPECIFICATION**

(45) Date of publication and mention of the grant of the patent:
**21.11.2012 Bulletin 2012/47**

(21) Application number: **06833453.1**

(22) Date of filing: **28.11.2006**

(51) Int Cl.:
***A61K 31/4427*** *(2006.01)* ***A61P 9/04*** *(2006.01)*
***A61P 9/10*** *(2006.01)* ***A61P 9/12*** *(2006.01)*
***A61P 13/12*** *(2006.01)* ***C07D 401/12*** *(2006.01)*

(86) International application number:
**PCT/JP2006/323649**

(87) International publication number:
**WO 2007/063821 (07.06.2007 Gazette 2007/23)**

(54) **ACID ADDITION SALT OF OPTICALLY ACTIVE DIHYDROPYRIDINE DERIVATIVE**
SÄUREADDITIONSSALZ EINES OPTISCH AKTIVEN DIHYDROPYRIDINDERIVAT
SEL D'ADDITION ACIDE DE DERIVE DE DIHYDROPYRIDINE OPTIQUEMENT ACTIF

(84) Designated Contracting States:
**AT BE BG CH CY CZ DE DK EE ES FI FR GB GR HU IE IS IT LI LT LU LV MC NL PL PT RO SE SI SK TR**

(30) Priority: **29.11.2005 JP 2005344256**

(43) Date of publication of application:
**27.08.2008 Bulletin 2008/35**

(73) Proprietor: **Ube Industries, Ltd.**
**Ube-shi, Yamaguchi 755-8633 (JP)**

(72) Inventors:
- **HAGIHARA, Masahiko**
  **Yamaguchi 755-8633 (JP)**
- **SHIMIZU, Motohisa**
  **Yamaguchi 755-8633 (JP)**
- **KOBAYASHI, Katsuhiro**
  **Tokyo 140-8710 (JP)**
- **YOSHIGAE, Yasushi**
  **Tokyo 140-8710 (JP)**

(74) Representative: **HOFFMANN EITLE**
**Patent- und Rechtsanwälte**
**Arabellastrasse 4**
**81925 München (DE)**

(56) References cited:
**EP-A1- 0 266 922** **EP-A1- 1 285 655**
**EP-A1- 1 577 309** **WO-A1-2004/067003**

- **KOBAYASHI T. ET AL: 'Novel 2-amino-1,4-dihydropyridine calcium antagonists. II. Synthesis and antihypertensive effects of 2-amino-1,4-dihydropyridine derivatives having N, N-dialkylaminoalkoxycarbonyl groups at 3- and/or 5-position' CHEMICAL & PHARMACEUTICAL BULLETIN vol. 43, no. 5, 1995, pages 797 - 817, XP003013676**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 961 751 B1

Printed by Jouve, 75001 PARIS (FR)

## Description

### TECHNICAL FIELD

The present invention relates to a specific acid addition salt of (R)-2-amino-1,4-dihydro-6-methyl-4-(3-nitrophenyl)-3,5-pyridinedicarboxylic acid 3-(1-diphenyl-methylazetidin-3-yl) ester 5-isopropyl ester having excellent calcium antagonistic effect, hypotensive effect, vasodilative effect, cardioprotective effect, antiarteriosclerotic effect, diuretic effect, nephropathy inhibitory effect and lipid peroxide generation inhibitory effect;
a pharmaceutical composition containing a specific acid addition salt of (R)-2-amino-1,4-dihydro-6-methyl-4-(3-nitrophenyl)-3,5-pyridinedicarboxylic acid 3-(1-diphenylmethylazetidin-3-yl) ester 5-isopropyl ester as an active ingredient, preferably a pharmaceutical composition for treating or preventing hypertension, heart disease, arteriosclerosis or nephropathy, more preferably a pharmaceutical composition for treating or preventing hypertension or heart disease, and most preferably a pharmaceutical composition for treating or preventing hypertension.

### BACKGROUND ART

(±)-2-Amino-1,4-dihydro-6-methyl-4-(3-nitrophenyl)-3,5-pyridinedicarboxylic acid 3-(1-diphenylmethylazetidin-3-yl) ester 5-isopropyl ester [hereinafter may be referred to as "compound (Ia)"] and (R)-2-amino-1,4-dihydro-6-methyl-4-(3-nitrophenyl)-3,5-pyridinedicarboxylic acid 3-(1-diphenylmethylazetidin-3-yl) ester 5-isopropyl ester [hereinafter may be referred to as "compound (I)"] which is an optical isomer of the compound (Ia) and having R-configuration that are dihydropyridine calcium antagonists are known to have pharmacological activities such as a calcium antagonistic effect and hypotensive effect and be useful as a medicine for treatment of hypertension or the like (see Patent Documents 1 to 4). Further, compounds having a calcium antagonistic effect are known to be useful as therapeutic agents for heart disease, arteriosclerosis or nephropathy [for example, (i) Goodman & Gilman's The pharmacological basis of therapeutics, chapter 32, p.767-774; (ii) Annual Report of Sankyo Research Laboratories, 2002, vol.54, p.1-64; (iii) The American Journal of Medicine, 1989, vol.86 (suppl 4A), p.27-32; and (iv) The American Journal of Hypertention, 1993, vol.6, p. 251S-259S].

Kobayashi et al. (Chemical & Pharmaceutical Bulletin, vol. 43, no. 5, 1995, pages 797-817) discloses racemic Azelnidipine as $(HCl)_2$ salts (Table 1, page 800, compound 44).

A further disclosure of racemic Azelnidipine $(HO)_2$ salt is EP0266922 (ibid, example 1).

A free form of the compound (I) can be obtained by optically resolving the compound (Ia) by the high-performance liquid chromatography (hereinafter may be referred to as "HPLC") method as an amorphous solid. Also, an acid addition salt of the compound (I) which can be obtained as a crystalline solid has not yet been known.

It is useful to find a compound having properties such as solubility, oral absorbability, concentration in blood and bioavailability (BA) superior to those of the free compound (I). It is also useful to find an acid addition salt of the compound (I) which can be obtained as a crystalline solid in order to supply a pharmaceutical compound having a certain quality in an industrial scale. EP 1 577 309 (Patent Document 3) discloses the (R)-enantiomer of Azelnidipine to be a pharmacologically more active one. It also suggests HCl and HBr salts of (R)-Azelnidipine.
Patent Document 1: JP 3-31715A
Patent Document 2: U.S. Patent No. 4,772,596, EP 0 266 922 A1 (Sankyo Co, Ube Industries)
Patent Document 3: JP 2004-217635A, EP 1 577 309 A1 (Sankyo Co, Ube Industries)
Patent Document 4: U.S. 2005/0272715A Patent Document 5: EP 1961 749 discloses (+/-) Azelnidipine $(HBr)_2$ salt

### DISCLOSURE OF THE INVENTION

### PROBLEMS TO BE SOLVED BY THE INVENTION

The present inventors have conducted extensive studies on acid addition salts of the compound (I) and have found that a specific acid addition salt of the compound (I) has excellent calcium antagonistic effect and hypotensive effect, for example, and is excellent as a pharmaceutical compound in terms of properties such as bioavailability, crystallinity and thermal stability, and is therefore useful as a medicine, in particular, a medicine for treating or preventing hypertension or the like. This finding has led to the completion of the present invention.

### MEANS TO SOLVE THE PROBLEMS

The present invention provides a specific acid addition salt of (R)-2-amino-1,4-dihydro-6-methyl-4-(3-nitrophenyl)-3,5-pyridinedicarboxylic acid 3-(1-diphenyl-methylazetidin-3-yl) ester 5-isopropyl ester having excellent calcium antagonistic effect, hypotensive effect, vasodilative effect, cardioprotective effect, antiarteriosclerotic effect, diuretic

effect, nephropathy inhibitory effect and lipid peroxide generation inhibitory effect;
a pharmaceutical composition containing the specific acid addition salt of (R)-2-amino-1,4-dihydro-6-methyl-4-(3-nitrophenyl)-3,5-pyridinedicarboxylic acid 3-(1-diphenylmethylazetidin-3-yl) ester 5-isopropyl ester as an active ingredient, preferably a pharmaceutical composition for treating or preventing hypertension, heart disease, arteriosclerosis or nephropathy (hereinafter also includes heart disease, arteriosclerosis or nephropathy caused by hypertension), more preferably a pharmaceutical composition for treating or preventing hypertension or heart disease, and most preferably a pharmaceutical composition for treating or preventing hypertension.

The present invention provides:

(1) A dihydrochloride dihydrate or dihydrobromide dihydrate of (R)-2-amino-1,4-dihydro-6-methyl-4-(3-nitrophenyl)-3,5-pyridinedicarboxylic acid 3-(1-diphenylmethylazetidin-3-yl) ester 5-isopropyl ester.
(2) A crystal of the dihydrochloride dihydrate of (R)-2-amino-1,4-dihydro-6-methyl-4-(3-nitrophenyl)-3,5-pyridinedicarboxylic acid 3-(1-diphenylmethylazetidin-3-yl) ester 5-isopropyl ester according to (1).
(3) The crystal according to which shows main d spacing peaks at 17, 7.1, 4.9, 4.3, 3.9 and 3.5 Å in a powder X-ray diffraction pattern obtained by irradiation with Cu Kα rays.
(4) A crystal the dihydrobromide dihydrate of (R)-2-amino-1,4-dihydro-6-methyl-4-(3-nitrophenyl)-3,5-pyridinedicarboxylic acid 3-(1-diphenylmethylazetidin-3-yl) ester 5-isopropyl ester according to (1).
(5) The crystal according to (4), which shows main d spacing peaks at 17, 4.0, 3.9, 3.5 and 3.2 Å in a powder X-ray diffraction pattern obtained by irradiation with Cu Kα rays.
(6) A pharmaceutical composition for treating or preventing hypertension, heart disease, arteriosclerosis or nephropathy, the pharmaceutical composition comprising the salt compound according to any one of claims 1 to 5 as an active ingredient.
(7) The pharmaceutical composition according to (6) for treating or preventing hypertension.
(8) Use of the salt compound according to any one of (1) to (5) for producing a pharmaceutical composition for treating or preventing hypertension, heart disease, arteriosclerosis or nephropathy.
(9) The use according to (8) for producing a pharmaceutical composition for treating or preventing hypertension.

In the present invention, (R)-2-amino-1,4-dihydro-6-methyl-4-(3-nitrophenyl)-3,5-pyridinedicarboxylic acid 3-(1-diphenylmethylazetidin-3-yl) ester 5-isopropyl ester is a compound having the following structural formula (I-1).

[Formula 1]

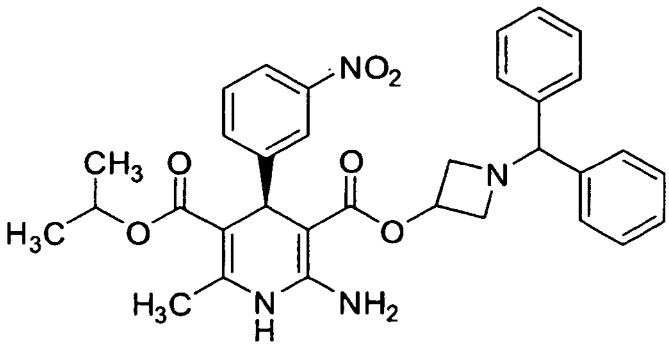

( I-1 )

In the present invention, the acid moiety in the acid addition salt of the compound (I) is a hydrochloric acid or hydrobromic acid, and most preferably hydrobromic acid.

In the present invention, the compound (I) has three basic groups (amino, azetidin-3-yl and dihydropyridyl groups). In the acid addition salt formed by the compound (I) and a monovalent, the molar ratio of the compound (I) to the acid [compound (I)/acid] is 1/2 in the case of the monovalent acid.

In the present invention, the hydrochloride of the compound (I) is a dihydrochloride dihydrate. The hydrobromide of the compound (I) is a dihydrobromide dihydrate.

In the present invention, the dehydrate acid addition salt of the compound (I) may form a crystal having a plurality of different internal structures and physicochemical properties (crystal polymorphism) depending on the reaction conditions and crystallization conditions. Such individual crystals or mixtures thereof at any ratio are included in the present invention. A crystalline solid and an amorphous solid may be mixed. Such a mixture at any ratio is included in the present invention. That is, the crystal of the present invention having a specific crystal form may contain a crystal having another crystal form or an amorphous solid. The content of the specific crystal form is preferably 50% or more,

more preferably 80% or more, still more preferably 90% or more, yet more preferably 93% or more, particularly preferably 95% or more, and most preferably 97% or more.

In the present invention, the crystal represents a solid having an internal structure three-dimensionally formed by regular repetition of constituent atoms (or groups of constituent atoms) and is distinguished from an amorphous solid not having such a regular internal structure. Whether or not a solid is a crystal can be examined by a crystallographically known method (such as powder X-ray crystallography and differential scanning calorimetry). For example, in powder X-ray crystallography of a solid using X-rays obtained by irradiation with Cu $K\alpha$ rays, the solid is determined to be a crystal when a specific peak is observed in its X-ray diffraction pattern, and the solid is determined to be amorphous when a specific peak is not observed. The solid is determined to be a crystal having a low degree of crystallinity when the peak can be read but is not clear (for example, broad); such a crystal having a low degree of crystallinity is also included in the crystal of the present invention.

In powder X-ray crystallography using Cu $K\alpha$ rays, a sample is usually irradiated with Cu $K\alpha$ rays (in which $K\alpha1$ and $K\alpha2$ rays are not separated). An X-ray diffraction pattern can be obtained by analyzing diffraction derived from $K\alpha$ rays, or alternatively can be obtained by analyzing only diffraction derived from $K\alpha1$ rays taken from diffraction derived from $K\alpha$ rays. In the present invention, the powder X-ray diffraction pattern obtained by irradiation with $K\alpha$ rays includes an X-ray diffraction pattern obtained by analyzing diffraction derived from $K\alpha$ rays and an X-ray diffraction pattern obtained by analyzing diffraction derived from $K\alpha1$ rays and is preferably an X-ray diffraction pattern obtained by analyzing diffraction derived from $K\alpha1$ rays.

In the following powder X-ray diffraction patterns of Figures 1 to 6, the vertical axis indicates a diffraction intensity [counts/seconds (cps)] and the horizontal axis indicates a diffraction angle $2\theta$ (°). The d spacing (Å) can be calculated by the formula $2d\sin\theta = n\lambda$ where n = 1. In the above formula, $K\alpha$ rays have a wavelength $\lambda$ of 1.54 Å and $K\alpha1$ rays have a wavelength $\lambda$ of 1.541 Å. Since the position and relative intensity of the d spacing may change depending on the measurement conditions and the like, identity of the crystal form should be recognized with reference to the entire spectral pattern appropriately even when the d spacing slightly differs.

The crystal of the dihydrochloride dihydrate of the compound (I) of the present invention is a crystal showing main d spacing peaks at 17, 7.1, 4.9, 4.3, 3.9 and 3.5 Å in a powder X-ray diffraction pattern obtained by irradiation with Cu $K\alpha$ rays which is shown in Figure 1, for example. Here, the main peak is a peak having a relative intensity of 78 or more when a peak of d spacing at 7.1 A has an intensity of 100.

The crystal of the dihydrobromide dihydrate of the compound (I) of the present invention is a crystal showing main d spacing peaks at 17, 4.0, 3.9, 3.5 and 3.2 Å in a powder X-ray diffraction pattern obtained by irradiation with Cu $K\alpha$ rays which is shown in Figure 2, for example. Here, the main peak is a peak having a relative intensity of 25 or more when a peak of d spacing at 17 Å has an intensity of 100.

In the present invention, heart disease includes angina pectoris. Heart disease, arteriosclerosis or nephropathy includes heart disease, arteriosclerosis or nephropathy caused by hypertension, respectively. Hypertension includes hypertension caused by heart disease, arteriosclerosis or nephropathy.

## EFFECTS OF THE INVENTION

The specific acid addition salt of (R)-2-amino-1,4-dihydro-6-methyl-4-(3-nitrophenyl)-3,5-pyridinedicarboxylic acid 3-(1-diphenylmethylazetidin-3-yl) ester 5-isopropyl ester of the present invention has excellent calcium antagonistic effect, hypotensive effect, vasodilative effect, cardioprotective effect, antiarteriosclerotic effect, diuretic effect, nephropathy inhibitory effect and lipid peroxide generation inhibitory effect and is excellent as a pharmaceutical compound in terms of properties such as physicochemical properties, thermal stability, storage and handling stability, residual solvent ratio, hygroscopicity, deliquescence, solubility, pharmacological properties, pharmacokinetic properties, oral absorbability, concentration in blood, bioavailability, pharmacokinetics, safety and toxicity. Therefore, the acid addition salt is useful as a medicine, preferably a medicine for treating or preventing hypertension, heart disease, arteriosclerosis or nephropathy, more preferably a medicine for treating or preventing hypertension or heart disease, and most preferably a medicine for treating or preventing hypertension. Further, the specific acid addition salt of the compound (I) of the present invention may have excellent properties in that the concentration in blood varies only slightly according to a change in intragastric pH and is difficult to be affected by the diet. Therefore, the acid addition salt is useful as a medicine for a warm-blooded animal, and preferably as a medicine for a human.

## BEST MODE FOR CARRYING OUT THE INVENTION

In the present invention, the acid addition salt of the compound (I) can be produced by the following method including:

(Step 1) dissolving the compound (I) in an inert solvent or water-containing inert solvent and adding an acid or an

aqueous solution or an inert solvent solution of an acid dropwise to the solution;
(Step 2) stirring the mixture at a certain temperature (preferably at room temperature) for a certain period of time; and
(Step 3) collecting the formed solid by filtration and drying the solid.

As necessary, it is possible to carry out before or after Step 2 one or more steps selected from the group consisting of the following steps:

(Step 4-1) adding seed crystals;
(Step 4-2) evaporating part of the solvent;
(Step 4-3) adding a poor solvent (an inert solvent in which the acid addition salt is insoluble); and
(Step 4-4) initiating or promoting precipitation of the crystals by providing mechanical stimulation such as ultrasonic stimulation or abrasion on the surface of the reaction vessel.

In Steps 1 and 2, water is preferably present or a hydrate of an acid is preferably used. In Step 1, an aqueous solution of an acid is preferably added dropwise to a solution of the compound (I) in an inert solvent.

The compound (I) used in the above production method can be produced according to the method described in Example 1 of JP 2004-217635A (U.S. 2005/0272715A). The compound (I) may be used as any of an isolated and purified product, a solid state crude reaction product and a solution of a crude reaction product.

The inert solvent used is not particularly limited insofar as it does not inhibit the reaction and allows the starting material to be dissolved therein to a certain extent. Examples of the solvent may include aliphatic hydrocarbons such as hexane, pentane, petroleum ether and cyclohexane; aromatic hydrocarbons such as benzene, toluene and xylene; halogenated hydrocarbons such as methylene chloride, chloroform, carbon tetrachloride, dichloroethane, chlorobenzene and dichlorobenzene; ethers such as diethyl ether, diisopropyl ether, dibutyl ether, butyl methyl ether, sec-butyl methyl ether, tert-butyl methyl ether, tetrahydrofuran, dioxane, dimethoxyethane and diethylene glycol dimethyl ether; ketones such as acetone, methyl ethyl ketone, methyl isobutyl ketone and cyclohexanone; esters such as ethyl acetate, propyl acetate and butyl acetate; nitriles such as acetonitrile, propionitrile, butyronitrile and isobutyronitrile; alcohols such as methanol, ethanol, 1-propanol, 2-propanol, 1-butanol, 2-butanol, 2-methyl-1-propanol and 2-methyl-2-propanol; amides such as formamide, dimethylformamide, dimethylacetamide, N-methyl-2-pyrrolidone and hexamethylphosphoric triamide; water; and mixtures thereof. Preferable examples of the solvent include ethers, ketones, esters, alcohols, water and mixtures thereof, more preferably tert-butyl methyl ether, acetone, ethyl acetate, 2-propanol and mixtures of these solvents and water, still more preferably a mixture of ethyl acetate and water, a mixture of acetone and water, or a mixture of 2-propanol and water, still further preferably a mixture of acetone and water or a mixture of 2-propanol and water, and most preferably a mixture of acetone and water. A suitable amount of water is preferably present in the reaction solution.

When the acid to be used is a monovalent acid, the amount of the acid used may be 0.4 to 10 mol per mol of the compound (I), for example, and is preferably 0.6 to 6 mol, and more preferably 0.8 to 5 mol.

When the acid to be used is a divalent acid, the amount of the acid used may be 0.2 to 10 mol per mol of the compound (I), for example, and is preferably 0.3 to 6 mol, and more preferably 0.4 to 4 mol.

When the acid to be used is a trivalent acid, the amount of the acid used may be 0.1 to 10 mol per mol of the compound (I), for example, and is preferably 0.2 to 6 mol, and more preferably 0.3 to 4 mol.

The concentration of the acid to be used in the aqueous solution or inert solvent solution may be 0.1 mol/l to saturation, for example, and is preferably 1 to 20 mol/l, and more preferably 3 to 15 mol/l.

The reaction temperature is usually -20°C to 150°C, preferably 0°C to 100°C, and more preferably 10°C to 60°C.

The reaction time varies according to the acid to be used, the solvent to be used, the reaction temperature or the like and is usually 5 minutes to 24 hours, preferably 10 minutes to 12 hours, and more preferably 20 minutes to 6 hours.

The formed solid may be isolated by filtration, centrifugation or a gradient method, for example. The isolated solid may be washed with an inert solvent (preferably an inert solvent used in the reaction) as necessary.

The isolated solid may be dried under reduced pressure usually at 20°C to 80°C, and preferably 30°C to 60°C. The drying time is usually a time until the weight becomes almost unchanged, and is preferably 30 minutes to 12 hours, and more preferably 1 to 6 hours. The solid may be dried in the presence of a drying agent such as a silica gel and/or calcium chloride as necessary.

The above reaction conditions are preferably conditions where hydrolysis reaction of two ester groups in the compound (I) does not occur.

The acid addition salt of the compound (I) of the present invention used as a medicine can be administered as is (as a bulk powder). Alternatively, the acid addition salt can be administered orally as a preparation such as tablets, capsules, granules, powder or syrup produced by mixing with an appropriate pharmacologically acceptable excipient or diluent, for example, or parenterally as a preparation such as an injection or suppository similarly produced (preferably orally).

These preparations are produced by a known method using additives such as an excipient, a lubricant, a binder, a disintegrator, an emulsifier, a stabilizer, a corrigent and/or a diluent.

The excipient may be an organic excipient or an inorganic excipient, for example. Examples of the organic excipient may include sugar derivatives such as lactose, sucrose, glucose, mannitol and sorbitol; starch derivatives such as corn starch, potato starch, α-starch and dextrin; cellulose derivatives such as crystalline cellulose; Gum Arabic; dextran; and pullulan. Examples of the inorganic excipient may include light silicic anhydride; and sulfates such as calcium sulfate.

Examples of the lubricant may include stearic acid; stearic acid metal salts such as calcium stearate and magnesium stearate; talc; colloidal silica; waxes such as beeswax and spermaceti; boric acid; adipic acid; sulfates such as sodium sulfate; glycol; fumaric acid; sodium benzoate; D,L-leucine; lauryl sulfates such as sodium lauryl sulfate and magnesium lauryl sulfate; silicic acids such as silicic anhydride and silicic acid hydrate; and the starch derivatives for the aforementioned excipient.

Examples of the binder may include hydroxypropyl cellulose, hydroxypropylmethyl cellulose, polyvinylpyrrolidone, polyethylene glycol and the compounds shown for the aforementioned excipient.

Examples of the disintegrator include cellulose derivatives such as low-substituted hydroxypropyl cellulose, carboxymethyl cellulose, calcium carboxymethyl cellulose and internally crosslinked sodium carboxymethyl cellulose; crosslinked polyvinylpyrrolidone; and chemically modified starches and celluloses such as carboxymethyl starch and sodium carboxymethyl starch.

Examples of the emulsifier may include colloidal clays such as bentonite and bee gum; anionic surfactants such as sodium lauryl sulfate and calcium stearate; cationic surfactants such as benzalkonium chloride; and nonionic surfactants such as polyoxyethylene alkyl ether, polyoxyethylene sorbitan fatty acid ester and sucrose fatty acid ester.

Examples of the stabilizer may include p-hydroxybenzoic acid esters such as methylparaben and propyl-paraben; alcohols such as chlorobutanol, benzyl alcohol and phenylethyl alcohol; benzalkonium chloride; phenols such as phenol and cresol; thimerosal; dehydroacetic acid; and sorbic acid.

Examples of the corrigent may include sweeteners such as sodium saccharin and aspartame; acidulants such as citric acid, malic acid and tartaric acid; and flavors such as menthol, lemon extract and orange extract.

The diluent may be a compound usually used as a diluent. Examples of the diluent may include lactose, mannitol, glucose, sucrose, calcium sulfate, hydroxypropyl cellulose, microcrystalline cellulose, water, ethanol, polyethylene glycol, propylene glycol, glycerol, starch, polyvinylpyrrolidones and mixtures thereof.

The dose of the acid addition salt of the compound (I) of the present invention may vary according to the conditions such as the symptom, age and body weight of the patient. The acid addition salt can be orally administered at 0.002 mg/kg (preferably 0.01 mg/kg) as the lower limit to 10 mg/kg (preferably 5 mg/kg) as the upper limit, or parenterally administered at 0.0002 mg/kg (preferably 0.001 mg/kg) as the lower limit to 10 mg/kg (preferably 5 mg/kg) as the upper limit to an adult in one to six times per day in response to the symptom.

EXAMPLES

The present invention will be described in more detail below with reference to Examples, Reference Examples, Test Example and Preparation Examples; however, the scope of the present invention is not limited thereto. In the following Examples, (R)-2-amino-1,4-dihydro-6-methyl-4-(3-nitrophenyl)-3,5-pyridinedicarboxylic acid 3-(1-diphenylmethyl-azetidin-3-yl) ester 5-isopropyl ester can be produced according to the method described in Example 1 of JP 2004-217635A (U.S. 2005/0272715A).

(Example 1) (R)-2-Amino-1,4-dihydro-6-methyl-4-(3-nitrophenyl)-3,5-pyridinedicarboxylic acid 3-(1-diphenylmethyl-azetidin-3-yl) ester 5-isopropyl ester dihydrochloride dihydrate

To a solution of 8.74 g (15.0 mmol) of (R)-2-amino-1,4-dihydro-6-methyl-4-(3-nitrophenyl)-3,5-pyridinedicarboxylic acid 3-(1-diphenylmethylazetidin-3-yl) ester 5-isopropyl ester in 240 ml of ethyl acetate was added dropwise 2.57 ml (30.0 mmol) of 36 wt% hydrochloric acid at 25°C over 30 minutes. After completion of the dropwise addition, the reaction solution was further stirred for 30 minutes. The formed crude crystals were collected by filtration and then washed with 15 ml of ethyl acetate and dried under reduced pressure at 50°C for two hours to give 9.20 g (89%) of the title compound as a white powder.

$^1$H-NMR spectrum (DMSO-$d_6$, δppm) : 0.99(d;J=6Hz,3H), 1.19(d;J=6Hz,3H), 2.29(s,3H), 4.03-4.27(m,4H), 4.73-4.90 (m,2H), 4.93-5.31(m,1H), 5.68-6.00(m,1H), 6.94(brs,2H), 7.33-7.74(m,12H), 7.92-8.03(m,2H), 9.18-9.30(m,1H) 12.53-12.81 (m, 1H) .

The powder X-ray diffraction pattern of this compound is shown in Figure 1. This compound had specific peaks in the X-ray diffraction pattern and was a crystalline solid.

This compound can also be produced using tert-butyl methyl ether as a solvent.

(Example 2) (R)-2-Amino-1,4-dihydro-6-methyl-4-(3-nitrophenyl)-3,5-pyridinedicarboxylic acid 3-(1-diphenylmethyl-azetidin-3-yl) ester 5-isopropyl ester dihydrobromide dihydrate

(2A) [Reaction solvent: acetone]

To a solution of 2.91 g (5.00 mmol) of (R)-2-amino-1,4-dihydro-6-methyl-4-(3-nitrophenyl)-3,5-pyridinedicarboxylic acid 3-(l-diphenylmethylazetidin-3-yl) ester 5-isopropyl ester in 7 ml of acetone was added dropwise a solution of 1.16 ml (10.0 mmol) of 47 wt% hydrobromic acid at 25°C over 30 minutes. After completion of the dropwise addition, the reaction solution was further stirred for 30 minutes. The formed crude crystals were collected by filtration and then washed with 7 ml of acetone and dried under reduced pressure at 60°C for two hours to give 2.68 g (69%) of the title compound as a white powder.
$^1$H-NMR spectrum (DMSO-$d_6$, δppm): 0.99(d;J=6Hz,3H), 1.19(d;J=6Hz,3H), 2.29(s,3H), 4.01-4.38(m,4H), 4.75-4.89(m, 2H), 4.96-5.23(m,1H), 5.78-6.08(m,1H), 6.85(brs,2H), 7.36-7.70(m,12H), 7.93-8.04(m,2H), 8.99(brs,1H), 11.16-11.61 (m,1H).
The powder X-ray diffraction pattern of this compound is shown in Figure 2. This compound had specific peaks in the X-ray diffraction pattern and was a crystalline solid.

(2B) [Reaction solvent: 2-propanol]

To a solution of 2.91 g (5.00 mmol) of (R)-2-amino-1,4-dihydro-6-methyl-4-(3-nitrophenyl)-3,5-pyridinedicarboxylic acid 3-(1-diphenylmethylazetidin-3-yl) ester 5-isopropyl ester in 30 ml of 2-propanol was added dropwise a solution of 1.16 ml (10.0 mmol) of 47 wt% hydrobromic acid at 25°C over 30 minutes. After completion of the dropwise addition, the reaction solution was further stirred for 30 minutes. The formed crude crystals were collected by filtration and then washed with 10 ml of 2-propanol and dried under reduced pressure at 60°C for two hours to give 2.68 g (69%) of the title compound as a white powder.
The $^1$H-NMR spectrum and the powder X-ray diffraction pattern of this compound are the same as those of the compound of Example (2A).

(2C) [Reaction solvent: ethyl acetate]

To a solution of 5.83 g (10.0 mmol) of (R)-2-amino-1,4-dihydro-6-methyl-4-(3-nitrophenyl)-3,5-pyridinedicarboxylic acid 3-(1-diphenylmethylazetidin-3-yl) ester 5-isopropyl ester in 160 ml of ethyl acetate was added dropwise a solution of 2.31 ml (20.0 mmol) of 47 wt% hydrobromic acid at 25°C over 30 minutes. After completion of the dropwise addition, the reaction solution was further stirred for 30 minutes. The formed crude crystals were collected by filtration and then washed with 30 ml of ethyl acetate and dried under reduced pressure at 50°C for two hours to give 6.98 g (89%) of the title compound as a white powder.
The $^1$H-NMR spectrum and the powder X-ray diffraction pattern of this compound are the same as those of the compound of Example (2A).
This compound can also be produced using tert-butyl methyl ether as a solvent.

(Comparative Example 1) (R)-2-Amino-1,4-dihydro-6-methyl-4-(3-nitrophenyl)-3,5-pyridinedicarboxylic acid 3-(1-diphenylmethylazetidin-3-yl) ester 5-isopropyl ester disulfate dihydrate

To a solution of 874 mg (1.50 mmol) of (R)-2-amino-1,4-dihydro-6-methyl-4-(3-nitrophenyl)-3,5-pyridinedicarboxylic acid 3-(1-diphenylmethylazetidin-3-yl) ester 5-isopropyl ester in 6 ml of ethyl acetate was added dropwise an aqueous sulfuric acid solution [0.176 ml (3.30 mmol) of conc. sulfuric acid/0.178 ml (9.90 mmol) of water] at 25°C over 30 minutes. After completion of the dropwise addition, the reaction solution was further stirred for 30 minutes. The formed crude crystals were collected by filtration and then washed with 2 ml of ethyl acetate and dried under reduced pressure at 50°C for two hours to give 861 mg (70%) of the title compound as a white powder.
$^1$H-NMR spectrum (DMSO-$d_6$, δppm): 1.00(d;J=6Hz,3H), 1.19(d;J=6Hz,3H), 2.28(s,3H), 4.03-4.43(m,4H), 4.73-4.89(m, 2H), 4.96-5.18(m,1H), 5.74-6.00(m,1H), 6.83(brs,2H), 7.36-7.66(m,12H), 7.93-8.04(m,2H), 8.93(brs,1H), 10.96-11.40 (m,1H).
The powder X-ray diffraction pattern of this compound is shown in Figure 3. This compound obtained under the conditions in the presence of water had specific peaks in the X-ray diffraction pattern and was a crystalline solid.

(Comparative Example 2) (R)-2-Amino-1,4-dihydro-6-methyl-4-(3-nitrophenyl)-3,5-pyridinedicarboxylic acid 3-(1-diphenylmethylazetidin-3-yl) ester 5-isopropyl ester monosulfate

A solution of 3.96 g (6.80 mmol) of (R)-2-amino-1,4-dihydro-6-methyl-4-(3-nitrophenyl)-3,5-pyridinedicarboxylic acid 3-(1-diphenylmethylazetidin-3-yl) ester 5-isopropyl ester in 12 ml of tert-butyl methyl ether was added dropwise 0.50 ml (9.38 mmol) of conc. sulfuric acid at 25°C over 30 minutes. After completion of the dropwise addition, the reaction solution was further stirred for two hours. The formed crude crystals were collected by filtration and then washed with 2 ml of tert-butyl methyl ether and dried under reduced pressure at 45°C for two hours to give 3.06 g (66%) of the title compound as a white powder.
$^1$H-NMR spectrum (DMSO-$d_6$, δppm): 1.00(d;J=6Hz,3H), 1.18(d;J=6Hz,3H), 2.28(s,3H), 4.01-4.41(m,4H), 4.75-4.90(m, 2H), 4.95-5.18(m,1H), 5.66-5.95(m,1H), 6.82(brs,2H), 7.36-7.66(m,12H), 7.92-8.04(m,2H), 8.89(brs,1H), 10.93-11.35 (m,1H).
The powder X-ray diffraction pattern of this compound is shown in Figure 4. This compound obtained under the conditions in the absence of water did not have a specific peak in the X-ray diffraction pattern and was an amorphous solid.
The results of Comparative Examples 1 and 2 showed that a production method using an aqueous acid solution is useful for obtaining the crystalline acid addition salt of the compound (I).

(Comparative Example 3) (R)-2-Amino-1,4-dihydro-6-methyl-4-(3-nitrophenyl)-3,5-pyridinedicarboxylic acid 3-(1-diphenylmethylazetidin-3-yl) ester 5-isopropyl ester dimethanesulfonate

To a solution of 4.66 g (8.00 mmol) of (R)-2-amino-1,4-dihydro-6-methyl-4-(3-nitrophenyl)-3,5-pyridinedicarboxylic acid 3-(1-diphenylmethylazetidin-3-yl) ester 5-isopropyl ester in 64 ml of ethyl acetate was added dropwise 1.04 ml (16.0 mmol) of methanesulfonic acid at 25°C over 30 minutes. After completion of the dropwise addition, the reaction solution was further stirred for 75 minutes. The formed crude crystals were collected by filtration and then washed with 25 ml of ethyl acetate and dried under reduced pressure at 60°C for four hours to give 6.03 g (97%) of the title compound as a white powder.
$^1$H-NMR spectrum (DMSO-$d_6$, δppm): 1.00(d;J=6Hz,3H), 1.18(d;J=6Hz,3H), 2.28(s,3H), 2.37(s,6H), 4.03-4.41(m,4H), 4.75-4.89(m,2H), 4.97-5.16(m,1H), 5.73-5.99(m,1H), 6.83(brs,2H), 7.37-7.66(m,12H), 7.94-8.03(m,2H), 8.94(brs,1H), 10.96-11.42(m,1H).
The powder X-ray diffraction pattern of this compound is shown in Figure 5. This compound had specific peaks in the X-ray diffraction pattern and was a crystalline solid.

(Comparative Example 4) (R)-2-Amino-1,4-dihydro-6-methyl-4-(3-nitrophenyl)-3,5-pyridinedicarboxylic acid 3-(1-diphenylmethylazetidin-3-yl) ester 5-isopropyl ester

[Compound (I)]

The title compound was produced according to the method described in Example 1 of JP 2004-217635A (U.S. 2005/0272715A).
The powder X-ray diffraction pattern of the obtained compound is shown in Figure 6. This compound did not have a specific peak in the X-ray diffraction pattern and was an amorphous solid.

(Test Example 1) Thermal stability test

The test compound was placed in a glass bottle and allowed to stand under the following conditions (1) and (2). After a certain period of time had elapsed, the residual rate of the active ingredient [compound (I)] in the test compound was measured by high performance liquid chromatography.
(1): Closed state, 60°C;
(2): Non-closed state, 40°C, humidity 75%.

The measurement conditions in high performance liquid chromatography are as follows.
Column: L-column ODS [4.6 mm x 250 mm, manufactured by Chemicals Evaluation and Research Institute, Japan]
Mobile layer: acetonitrile/22 mM potassium dihydrogenphosphate buffer/methanol = 455/350/195 (V/V/V) (adjusted

to pH 5.5 by phosphoric acid)
Flow rate: 1 ml/min
Column temperature: 40°C
Detection wavelength: 220 nm

The residual rate (%) was calculated by the following formula.
$$\text{The residual rate (\%)} = [1 - (\text{sum of peak area percentages of impurities and decomposed products})] \times 100$$

The results under the above conditions (1) and (2) are shown in Tables 1 and 2, respectively.
[Table 1] [Condition (1)]

| Test compound No. | Time: | Residual rate of Compound (I) (%) 0 | 1 week | 1 month | 3 months |
|---|---|---|---|---|---|
| Example 1 | | 99.4 | 96.6 | 95.3 | 92.0 |
| Example 2A | | 99.8 | 99.8 | 99.7 | 99.7 |
| Example 2C | | 99.1 | 95.0 | 94.2 | 94.5 |
| Comparative Example 1 | | 99.3 | 68.0 | 0.4 | |
| Comparative Example 2 | | 93.1 | 25.0 | 24.3 | |
| Comparative Example 3 | | 99.3 | 93.2 | 90.0 | 84.1 |

As is clear from the results in Table 1, the compound of Comparative Example 2 that was an amorphous solid had extremely low stability, and the compound of Comparative Example 1 had extremely low stability although the compound was a crystalline solid. The compound of Comparative Example 3 did not have high stability although the compound was a crystalline solid. In contrast, the compounds of Examples 1 and 2 (2A and 2C) of the present invention were crystalline solids and had excellent stability.
[Table 2] [Condition (2)]

| Test compound No. | Time: | Residual rate of Compound (I) (%) 0 | 1 week | 1 month | 3 months |
|---|---|---|---|---|---|
| Example 1 | | 99.4 | 97.8 | 96.0 | 94.1 |
| Example 2A | | 99.8 | 99.7 | 99.6 | 99.4 |
| Example 2C | | 99.1 | 95.5 | 92.1 | 90.6 |
| Comparative Example 1 | | 99.3 | 96.8 | 84.4 | |
| Comparative Example 2 | | 93.1 | 8.9 | | |
| Comparative Example 3 | | 99.3 | 0.1 | 0.0 | |

As is clear from the results in Table 2, the compound of Comparative Example 2 that was an amorphous solid had extremely low stability, and the compound of Comparative Example 3 had low stability although the compound was a crystalline solid. Also, the compound of Comparative Example 1 did not have high stability although the compound was a crystalline solid. In contrast, the compounds of Examples 1 and 2 (2A and 2C) of the present invention were crystalline solids and had excellent stability.
As is clear from the results in Tables 1 and 2, whereas there was no compound which showed excellent stability under the conditions of both of (1) and (2) among the compounds of Comparative Examples 1 to 3, the compounds of Examples 1 and 2 (2A and 2C) of the present invention each showed excellent stability under the conditions of both of (1) and (2).
The results in Tables 1 and 2 show that not all acid addition salts of the compound (I) that can be obtained as

crystalline solids have excellent thermal stability and the specific acid addition salts of the compound (I) of the present invention have thermal stability superior to those of other acid addition salts.

(Test Example 2) Absorbability test in dog

(1) Method

Ranitidine (2.5 mg/kg) was intramuscularly injected to the posterior region of thigh of a fasted male beagle (body weight: about 10 kg, n = 6) to make the intragastric pH acidic 30 minutes before, immediately before and 30 minutes after administration of the test compound. The test compound was suspended in a 0.5% methyl cellulose solution at a dose of 10 mg/body and orally administered. About 3 ml of blood was collected from the median antebrachial vein using a heparin-treated glass syringe 0.5, 1, 2, 3, 4, 6, 8 and 24 hours after the administration. The collected blood was centrifuged to obtain plasma which was cryopreserved at -20°C until measurement of the concentration of the test compound. One hundred μl of thawed plasma was mixed with 300 μl of an internal standard solution [a methanol solution of the $d_3$-compound (Ia), 5 ng/ml] and centrifuged. The supernatant was filtered through a filter and the filtrate was analyzed by LC/MS/MS.

A calibration curve was prepared by the same operation as above using each 100 μl of a calibration curve standard solution [a methanol solution of the compound (Ia), 0.5-400 ng/ml] which had been adjusted to respective concentrations in place of 100 μl of dog plasma.

The LC/MS/MS analysis conditions are shown below.

[MS/MS]

System: API3000 LC/MS/MS System (manufactured by Applied Biosystems)
Ion source: TurboIonSpray
Turbo heater gas: Air, 425°C, 7 1/min
Nebulizer gas: Air, 40 psi, 0.92 1/min
Curtain gas: $N_2$, 0.95 1/min
Orifice voltage: Compound (Ia) 101 V
$d_3$-Compound (Ia) 96 V
Ion spray voltage: 5000 V
Collision gas: $N_2$
Collision energy: Compound (Ia) 33 V
$d_3$-Compound (Ia) 37 V
Measurement mode: Positive/MRM
Monitor ion: Compound (Ia) m/z 583 -> 167
$d_3$-Compound (Ia) m/z 586 -> 167

[HPLC]

System: LC-10Avp (manufactured by Shimadzu Corporation)
Column: L-column ODS 1.5 mm I.D. x 150 mm (manufactured by Chemicals Evaluation and Research Institute, Japan)
Mobile phase: Acetonitrile/water/1M ammonium acetate aqueous solution/formic acid (60/40/0.1/0.05)
Flow rate: 0.2 ml/min
Column temperature: 40°C
Injection amount: 2 μl

(2) Results

The area under the concentration in plasma-time curve ($AUC_{0-24h}$) and the maximum concentration in plasma (Cmax) as pharmacokinetic parameters indicative of drug absorbability were calculated from the results obtained according to the aforementioned method using the compounds of Example 1, Example 2 (2C) and Comparative Example 4 as test compounds. The results are shown in Table 3.

[Table 3]

| Test compound No. | $AUC_{0-24h}$ [ng.h/ml] | Cmax [ng/ml] |
|---|---|---|
| Example 1 | 1660 | 168 |
| Example 2C | 1929 | 230 |
| Comparative Example 4 | 1497 | 161 |

The compound of the present invention [particularly the compound of Example 2] had bioavailability and concentration in blood ($AUC_{0-24h}$ and Cmax) superior to those of the compound of Comparative Example 4 [Compound (I)] which is a free form.

(Test Example 3) Calcium channel receptor binding test using rat cerebral cortex membrane fraction

A rat cerebral cortex membrane fraction was used as a source of the L-type calcium channel, and $^3$H-(+)-isradipine was used as a ligand of the L-type calcium channel. The membrane fraction (5.0 mg protein/ml), $^3$H-(+)-isradipine (0.5 nM) and the test compound were reacted in a tris-(hydroxymethyl)aminomethane hydrochloride (Tris-HCl; 50 mM, pH 7.4) buffer at room temperature for 60 minutes. Then, $^3$H-(+)-isradipine bound to the membrane fraction was measured using a liquid scintillation counter. The count in the presence of unlabeled nitrendipine (non-specific binding amount) was subtracted from the measured count to calculate the specific binding amount. The relation of the specific binding concentration and the binding inhibition rate for each compound was applied to a logit-log model to calculate the $IC_{50}$ value (50% inhibitory concentration of specific binding; nM) and the Ki value (dissociation constant; nM).

The compounds of Examples 1 and 2 had a Ki value of 1.4 to 2.0 nM each. The specific acid addition salt of the compound (I) of the present invention has an excellent calcium channel receptor antagonistic effect and is useful as a medicine for treating or preventing hypertension, heart disease, arteriosclerosis or nephropathy.

Test Example 3 may also be carried out using porcine myocardium microsome as a source of the L-type calcium channel.

(Test Example 4) Hypotensive effect test in hypertensive rat

The test compound was orally administered to a male spontaneously hypertensive rat without anaesthesia, and the blood pressure was measured by telemetry method every five minutes over 24 hours. The test compound was suspended in a 0.5% methylcellulose solution and administered to the rat. The area from the time of administration to 24 hours after the administration was calculated by the trapezoid method from the hypoglycemic rates at individual measurement points to determine the hypoglycemic rate area value (%·hr).

The compounds of Examples 1 and 2 had a hypoglycemic rate area value of 127 to 132 (%·hr) each. The specific acid addition salt of the compound (I) of the present invention has an excellent hypoglycemic effect and is useful as a medicine for treating or preventing hypertension or the like.

(Preparation Example 1) Capsules

Powders of the example compound (10.0 mg), lactose (168.7 mg), corn starch (70.0 mg) and magnesium stearate (1.3 mg) (250 mg in total) are mixed and allowed to pass through a 60-mesh sieve. Then, the resulting powder was put in No. 2 gelatin capsules to prepare capsules.

(Preparation Example 2) Tablets

Powders of the example compound (10.0 mg), lactose (149.0 mg), corn starch (40.0 mg) and magnesium stearate (1.0 mg) (200 mg in total) are mixed and tableted by a tableting machine to prepare tablets having a weight of 200 mg each.

INDUSTRIAL APPLICABILITY

The specific acid addition salt of (R)-2-amino-1,4-dihydro-6-methyl-4-(3-nitrophenyl)-3,5-pyridinedicarboxylic acid 3-(1-diphenylmethylazetidin-3-yl) ester 5-isopropyl ester of the present invention has excellent calcium antagonistic

effect, hypotensive effect, vasodilative effect, cardioprotective effect, antiarteriosclerotic effect, diuretic effect, nephropathy inhibitory effect and lipid peroxide generation inhibitory effect and is excellent as a pharmaceutical compound in terms of properties such as physicochemical properties, thermal stability, storage and handling stability, residual solvent ratio, hygroscopicity, deliquescence, solubility, pharmacological properties, pharmacokinetic properties, oral absorbability, concentration in blood, bioavailability, pharmacokinetics, safety and toxicity. Therefore, the acid addition salt is useful as a medicine, preferably a medicine for treating or preventing hypertension, heart disease, arteriosclerosis or nephropathy, more preferably a medicine for treating or preventing hypertension or heart disease, and most preferably a medicine for treating or preventing hypertension. Further, the specific acid addition salt of the compound (I) of the present invention may have excellent properties in that the concentration in blood varies only slightly according to a change in intragastric pH and is difficult to be affected by the diet. Therefore, the acid addition salt is useful as a medicine for a warm-blooded animal, and preferably as a medicine for a human.

BRIEF DESCRIPTION OF THE DRAWINGS

Figure 1 shows a powder X-ray diffraction pattern of (R)-2-amino-1,4-dihydro-6-methyl-4-(3-nitrophenyl)-3,5-pyridinedicarboxylic acid 3-(1-diphenylmethylazetidin-3-yl) ester 5-isopropyl ester dihydrochloride dihydrate obtained in Example 1;
Figure 2 shows a powder X-ray diffraction pattern of (R)-2-amino-1,4-dihydro-6-methyl-4-(3-nitrophenyl)-3,5-pyridinedicarboxylic acid 3-(1-diphenylmethylazetidin-3-yl) ester 5-isopropyl ester dihydrobromide dihydrate obtained in Example 2A;
Figure 3 shows a powder X-ray diffraction pattern of (R)-2-amino-1,4-dihydro-6-methyl-4-(3-nitrophenyl)-3,5-pyridinedicarboxylic acid 3-(1-diphenylmethylazetidin-3-yl) ester 5-isopropyl ester disulfate dihydrate obtained in Comparative Example 1;
Figure 4 shows a powder X-ray diffraction pattern of (R)-2-amino-1,4-dihydro-6-methyl-4-(3-nitrophenyl)-3,5-pyridinedicarboxylic acid 3-(1-diphenylmethylazetidin-3-yl) ester 5-isopropyl ester monosulfate obtained in Comparative Example 2;
Figure 5 shows a powder X-ray diffraction pattern of (R)-2-amino-1,4-dihydro-6-methyl-4-(3-nitrophenyl)-3,5-pyridinedicarboxylic acid 3-(1-diphenylmethylazetidin-3-yl) ester 5-isopropyl ester dimethanesulfonate obtained in Comparative Example 3; and
Figure 6 shows a powder X-ray diffraction pattern of (R)-2-amino-1,4-dihydro-6-methyl-4-(3-nitrophenyl)-3,5-pyridinedicarboxylic acid 3-(1-diphenylmethylazetidin-3-yl) ester 5-isopropyl ester obtained in Comparative Example 4.

**Claims**

1. A dihydrochloride dihydrate or dihydrobromide dihydrate of (R)-2-amino-1,4-dihydro-6-methyl-4-(3-nitrophenyl)-3,5-pyridinedicarboxylic acid 3-(1-diphenylmethyl-azetidin-3-yl) ester 5-isopropyl ester.

2. A crystal of the dihydrochloride dihydrate of (R)-2-amino-1,4-dihydro-6-methyl-4-(3-nitrophenyl)-3,5-pyridinedicarboxylic acid 3-(1-diphenylmethyl-azetidin-3-yl) ester 5-isopropyl ester according to claim 1.

3. The crystal according to claim 2, which shows main d spacing peaks at 17, 7.1, 4.9, 4.3, 3.9 and 3.5 Å in a powder X-ray diffraction pattern obtained by irradiation with Cu K$\alpha$ rays.

4. A crystal of the dihydrobromide dihydrate of (R)-2-amino-1,4-dihydro-6-methyl-4-(3-nitrophenyl)-3,5-pyridinedicarboxylic acid 3-(1-diphenylmethyl-azetidin-3-yl) ester 5-isopropyl ester according to claim 1.

5. The crystal according to claim 4, which shows main d spacing peaks at 17, 4.0, 3.9, 3.5 and 3.2 Å in a powder X-ray diffraction pattern obtained by irradiation with Cu K$\alpha$ rays.

6. A pharmaceutical composition for treating or preventing hypertension, heart disease, arteriosclerosis or nephropathy, the pharmaceutical composition comprising the salt compound according to any one of claims 1 to 5 as an active ingredient.

7. The pharmaceutical composition according to claim 6 for treating or preventing hypertension.

8. Use of the salt compound according to any one of claims 1 to 5 for producing a pharmaceutical composition for treating or preventing hypertension, heart disease, arteriosclerosis or nephropathy.

9. The use according to claim 8 for producing a pharmaceutical composition for treating or preventing hypertension.

**Patentansprüche**

1. Dihydrochlorid-dihydrat oder Dihydrobromid-dihydrat von (R)-2-Amino-1,4-dihydro-6-methyl-4-(3-nitrophenyl)-3,5-pyridindicarbonsäure-3-(1-diphenylmethyl-azetidin-3-yl)ester-5-isopropylester.

2. Kristall des Dihydrochlorid-dihydrats von (R)-2-Amino-1,4-dihydro-6-methyl-4-(3-nitrophenyl)-3,5-pyridindicarbonsäure-3-(1-diphenylmethyl-azetidin-3-yl)ester-5-isopropylester gemäss Anspruch 1.

3. Kristall gemäss Anspruch 2, bei dem die Hauptpeaks des Netzebenenabstands d bei 17, 7,1, 4,9, 4,3, 3,9 und 3,5 Å in einem Pulver-Röntgenbeugungsmuster, das durch Bestrahlung mit Cu Kα-Strahlen erhalten wird, beobachtet werden.

4. Kristall des Dihydrobromid-dihydrats von (R)-2-Amino-1,4-dihydro-6-methyl-4-(3-nitrophenyl)-3,5-pyridindicarbonsäure-3-(1-diphenylmethyl-azetidin-3-yl)ester-5-isopropylester gemäss Anspruch 1.

5. Kristall gemäss Anspruch 4, bei dem die Hauptpeaks des Netzebenenabstands d bei 17, 4,0, 3,9, 3,5 und 3,2 Å in einem Pulver-Röntgenbeugungsmuster, das durch Bestrahlung mit Cu Kα-Strahlen erhalten wird, beobachtet werden.

6. Pharmazeutische Zusammensetzung zur Behandlung oder Prävention von Bluthochdruck, Herzerkrankungen, Arteriosklerose oder Nephropathie, wobei die pharmazeutische Zusammensetzung die Salzverbindung gemäss irgendeinem der Ansprüche 1 bis 5 als wirksamen Bestandteil umfasst.

7. Pharmazeutische Zusammensetzung gemäss Anspruch 6 zur Behandlung oder Prävention von Bluthochdruck.

8. Verwendung der Salzverbindung gemäss irgendeinem der Ansprüche 1 bis 5 zur Herstellung einer pharmazeutischen Zusammensetzung zur Behandlung oder Prävention von Bluthochdruck, Herzerkrankungen, Arteriosklerose oder Nephropathie.

9. Verwendung gemäss Anspruch 8 zur Herstellung einer pharmazeutischen Zusammensetzung zur Behandlung oder Prävention von Bluthochdruck.

**Revendications**

1. Dichlorhydrate dihydraté ou dibromhydrate dihydraté d'ester de 5-isopropyle ester de 3-(1-diphénylméthyl-azétidin-3-yle) d'acide (R)-2-amino-1,4-dihydro-6-méthyl-4-(3-nitrophényl)-3,5-pyridinedicarboxylique.

2. Cristal du dichlorhydrate dihydraté d'ester de 5-isopropyle ester de 3-(1-diphénylméthyl-azétidin-3-yle) d'acide (R)-2-amino-1,4-dihydro-6-méthyl-4-(3-nitrophényl)-3,5-pyridinedicarboxylique selon la revendication 1.

3. Cristal selon la revendication 2, qui présente des pics principaux d'espacement d à 17, 7,1, 4,9, 4,3, 3,9 et 3,5 Å dans un diagramme de diffraction des rayons X sur poudre obtenu par irradiation avec des rayons Ka du Cu.

4. Cristal du dibromhydrate dihydraté d'ester de 5-isopropyle ester de 3-(1-diphénylméthyl-azétidin-3-yle) d'acide (R)-2-amino-1,4-dihydro-6-méthyl-4-(3-nitrophényl)-3,5-pyridinedicarboxylique selon la revendication 1.

5. Cristal selon la revendication 4, qui présente des pics principaux d'espacement d à 17, 4,0, 3,9, 3,5 et 3,2 Å dans un diagramme de diffraction des rayons X sur poudre obtenu par irradiation avec des rayons Ka du Cu.

6. Composition pharmaceutique destinée au traitement ou à la prévention de l'hypertension, d'une maladie cardiaque, de l'artériosclérose ou d'une néphropathie, la composition pharmaceutique comprenant le composé salin selon

l'une quelconque des revendications 1 à 5 en tant que principe actif.

**7.** Composition pharmaceutique selon la revendication 6 destinée au traitement ou à la prévention de l'hypertension.

**8.** Utilisation du composé salin selon l'une quelconque des revendications 1 à 5 pour la fabrication d'une composition pharmaceutique destinée à traiter ou prévenir l'hypertension, une maladie cardiaque, l'artériosclérose ou une néphropathie.

**9.** Utilisation selon la revendication 8 pour la fabrication d'une composition pharmaceutique destinée au traitement ou à la prévention de l'hypertension.

**Fig. 1**

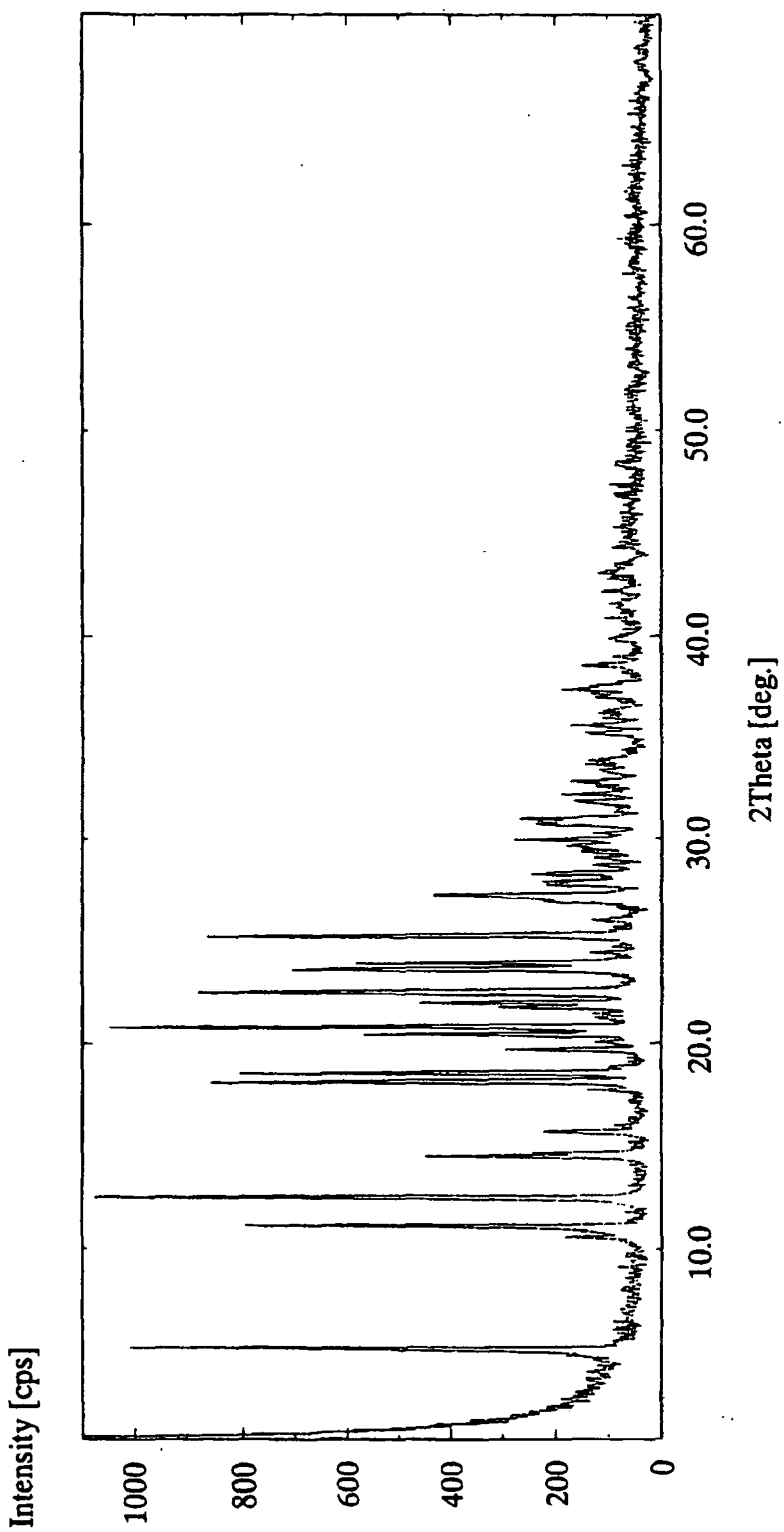
Intensity [cps]
1000
800
600
400
200
0
10.0
20.0
30.0
40.0
50.0
60.0
2Theta [deg.]

# Fig. 2

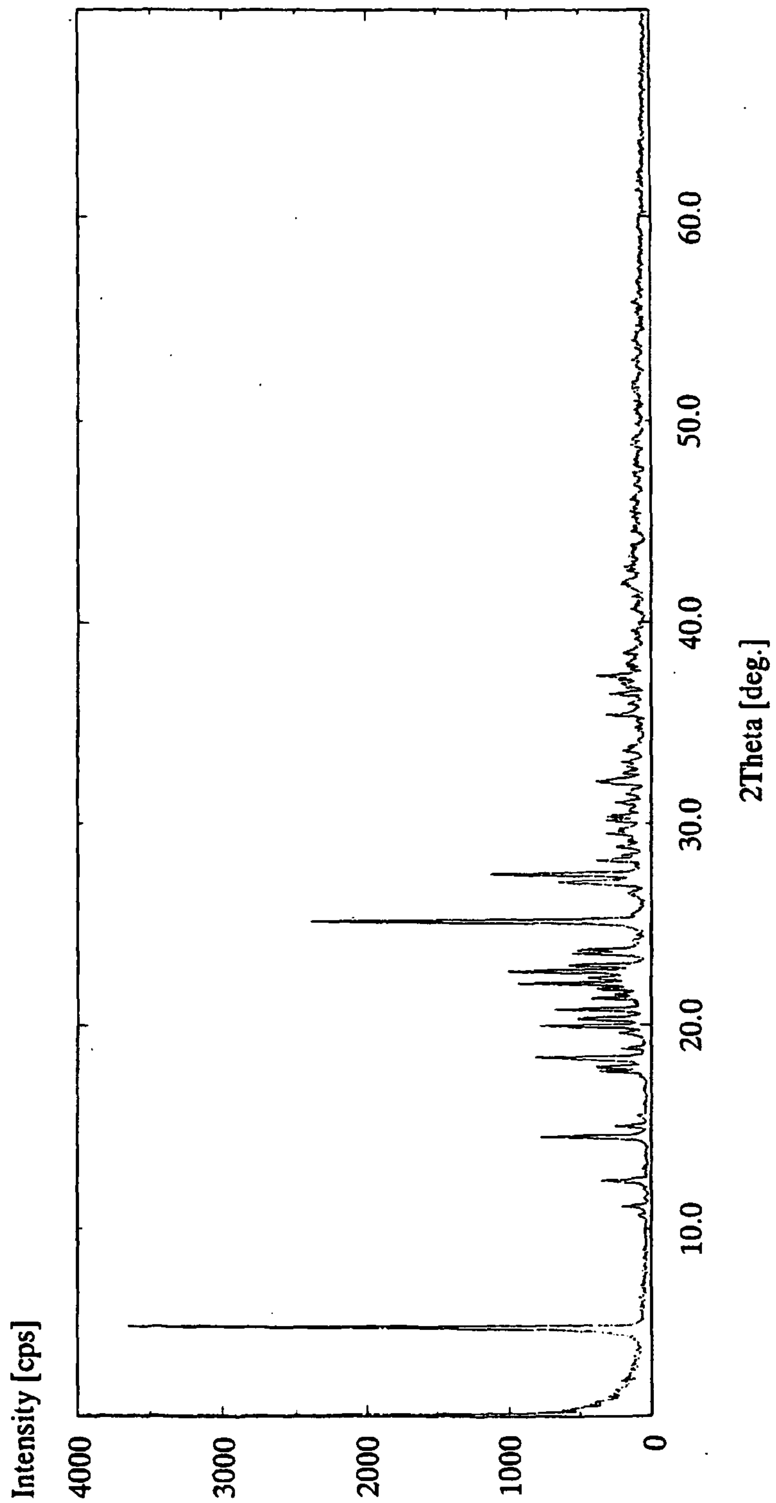
Intensity [cps]
4000
3000
2000
1000
0
10.0
20.0
30.0
40.0
50.0
60.0
2Theta [deg.]

Fig. 3

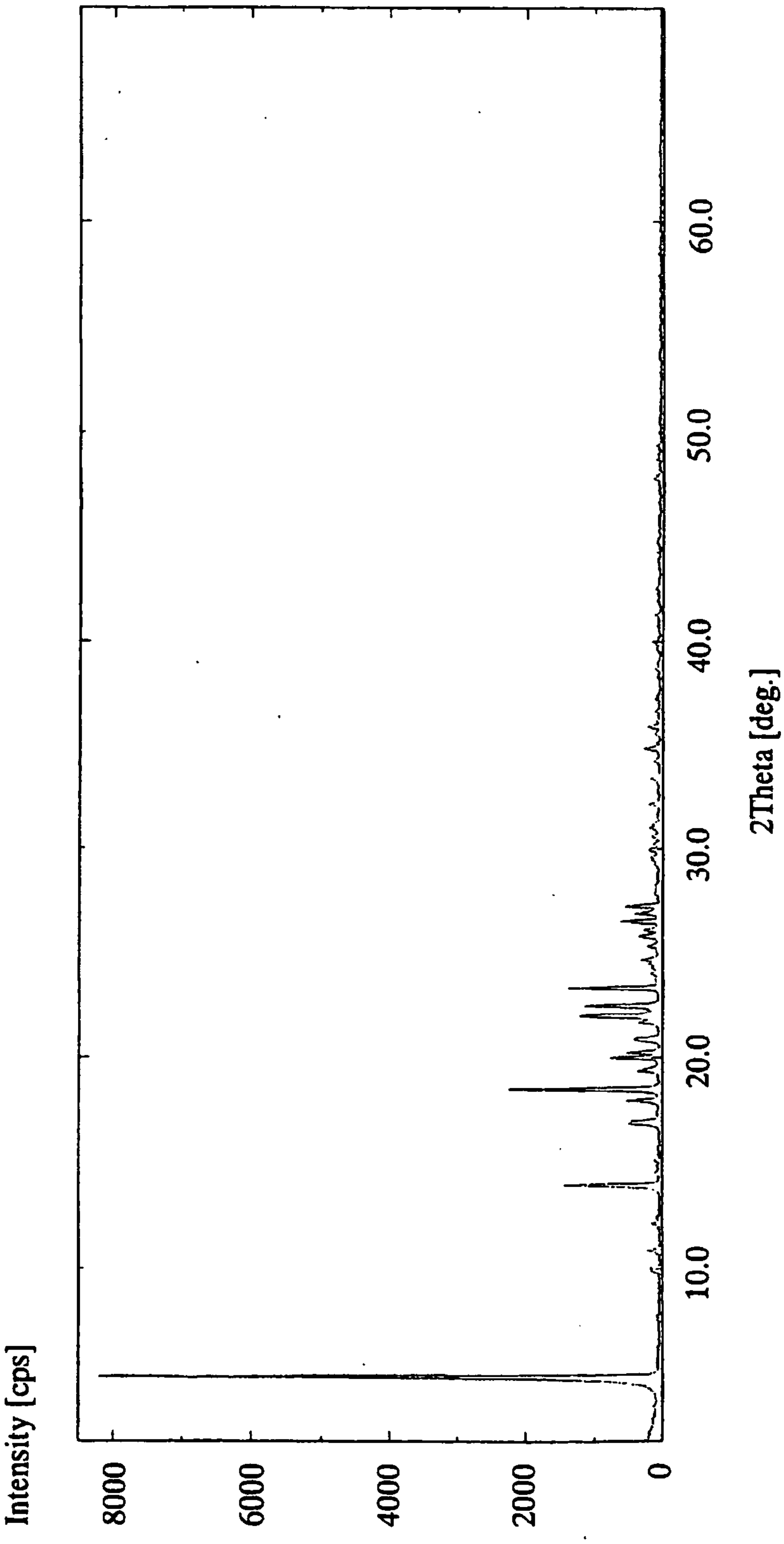
Intensity [cps]
8000
6000
4000
2000
0
10.0
20.0
30.0
40.0
50.0
60.0
2Theta [deg.]

# Fig. 4

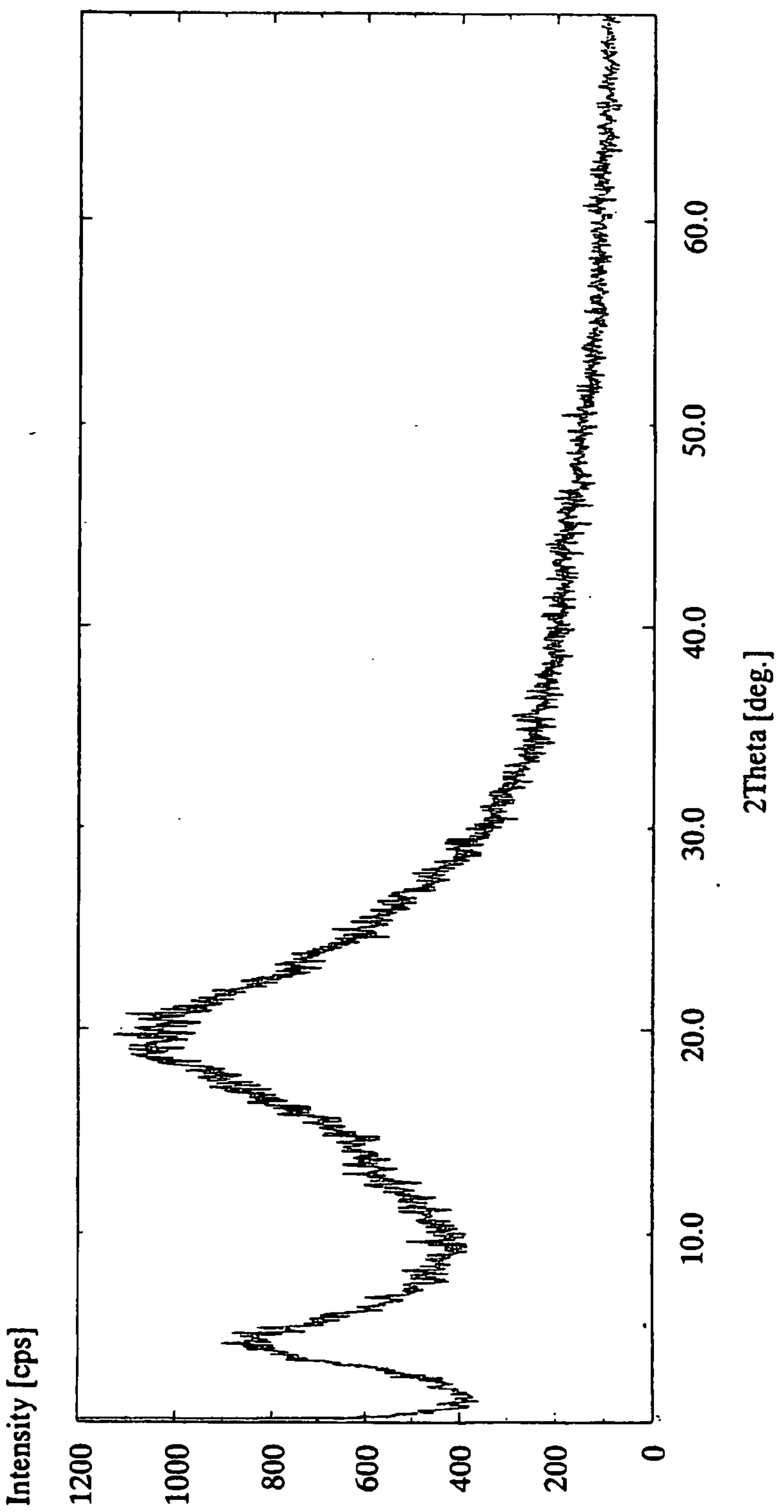
Intensity [cps]
1200
1000
800
600
400
200
0
10.0
20.0
30.0
40.0
50.0
60.0
2Theta [deg.]

# Fig. 5

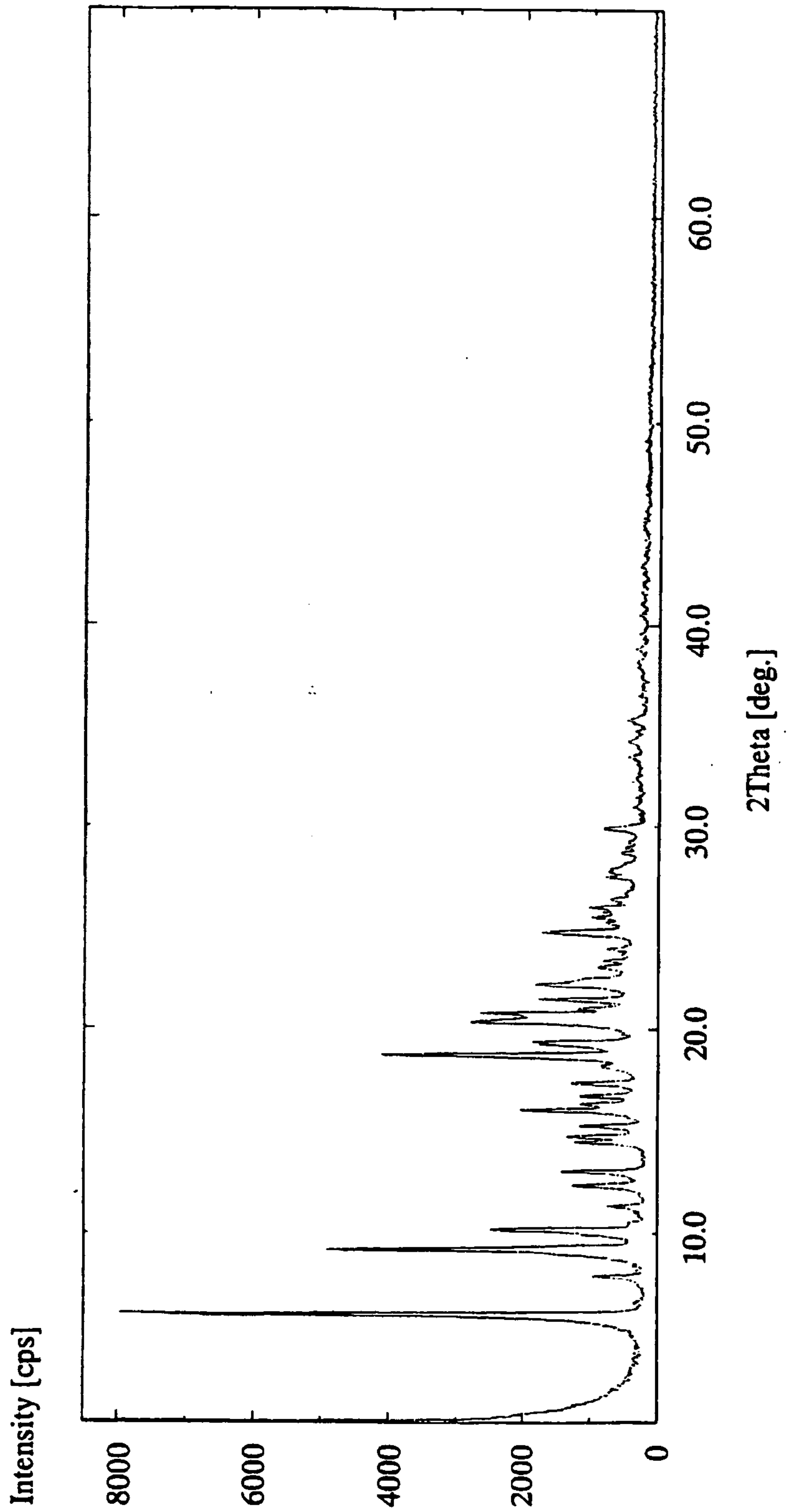
Intensity [cps]
8000
6000
4000
2000
0
10.0
20.0
30.0
40.0
50.0
60.0
2Theta [deg.]

# Fig. 6

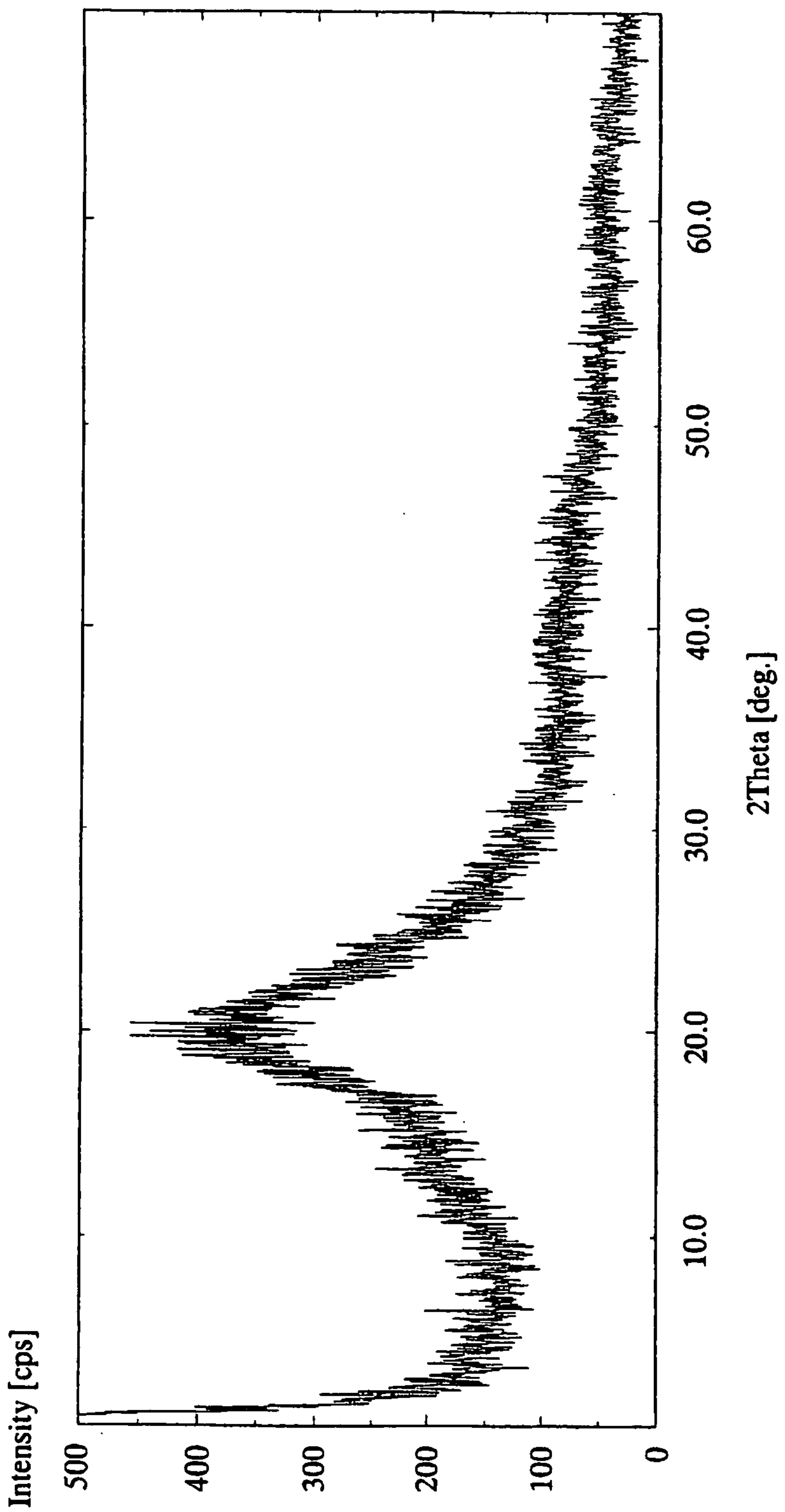
Intensity [cps]
500
400
300
200
100
0
10.0
20.0
30.0
40.0
50.0
60.0
2Theta [deg.]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0266922 A
- EP 1577309 A
- JP 3031715 A
- US 4772596 A
- EP 0266922 A1
- JP 2004217635 A
- EP 1577309 A1
- US 20050272715 A
- EP 1961749 A
- JP 2004 A
- JP 217635 A


### Non-patent literature cited in the description


- Goodman & Gilman's The pharmacological basis of therapeutics. 767-774
- *Annual Report of Sankyo Research Laboratories,* 2002, vol. 54, 1-64
- *The American Journal of Medicine,* 1989, vol. 86 (4A), 27-32
- *The American Journal of Hypertention,* 1993, vol. 6, 251S-259S
- **KOBAYASHI et al.** *Chemical & Pharmaceutical Bulletin,* 1995, vol. 43 (5), 797-817